(19)

Europäisches
Patentamt

European
Patent Office

Office européen
des brevets

(11) **EP 3 944 805 A1**

(12) **EUROPEAN PATENT APPLICATION**

(43) Date of publication:
**02.02.2022 Bulletin 2022/05**

(21) Application number: **20382695.3**

(22) Date of filing: **30.07.2020**

(51) International Patent Classification (IPC):
*A61B 1/24* (2006.01)     *A61B 90/20* (2016.01)
*A61B 90/00* (2016.01)     *A61C 3/00* (2006.01)
*G02B 21/00* (2006.01)     *A61B 1/00* (2006.01)
*G02B 25/00* (2006.01)     *A61C 19/00* (2006.01)
*G06T 7/00* (2017.01)

(52) Cooperative Patent Classification (CPC):
**A61B 1/00009; A61B 1/00087; A61B 1/00101;
A61B 1/247; A61B 90/361; G02B 21/0008;
G02B 21/0012;** A61B 90/20; A61B 2090/3616;
A61C 3/00; A61C 19/04; G02B 25/007

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Kreival Fast, S.L
08140 Barcelona (ES)**

(72) Inventors:
 • **Tinoco Vega, Sergio
  08140 BARCELONA (ES)**
 • **Alsina Font, Francesc
  08140 BARCELONA (ES)**

(74) Representative: **Herrero & Asociados, S.L.
  Cedaceros, 1
  28014 Madrid (ES)**

(54) **OPTICAL SYSTEM FOR SURGICAL PRACTICE AND METHOD FOR CARRYING OUT A SURGICAL PRACTICE WITH IMAGE MAGNIFICATION**

(57) The present invention relates to an optical system and a method for carrying out an image magnification surgical practice in which the optical system comprises a holding body (1) with a digital microscope; a set of tools (2) with means of attachment to the holding body (1) and an active tip (3); a display device (4) to show the image taken by the digital microscope once processed, the clamping body (1) comprising a first (1') and second (1") ends such that the first end (1') comprises the lens of the digital microscope and means of attachment to the tools (2) that allow the locking without relative movement of both elements to each other so that the active tip (3) is located at a length L known to the lens of the digital microscope .

FIG.1

EP 3 944 805 A1

## Description

## Object of the invention

The present invention is within the technical field of medicine or, more specifically, within the technical field that refers to surgical practices in which a surgeon, doctor, technician or specialist person performs on some part of the body of a patient a procedure that needs the help of an optical instrument

Still more specifically, the object of the present invention refers to an optical system capable of magnifying the image so that said surgeon, doctor, technician or specialist can carry out the appropriate surgical procedure in the safest and most efficient way possible, which can be framed within fields as diverse as dermatology, podiatry, dentistry, etc., and, in general, in any that requires an extension of the area of the patient's body on which said procedure is performed.

Likewise, the invention also refers, as the title itself indicates, to a method to carry out a surgical practice with image magnification in a comfortable and simple way for the aforementioned surgeon, doctor, technician or specialist person.

## Background of the invention

As it is known, the use of optical magnifying devices is today a widely extended practice, especially in medical or surgical procedures, where either in major surgeries or in minor procedures or operation, it is increasingly necessary and/or advisable to have a close-up of the work area, which is shown enlarged with the help of a projector screen, etc. Thus, there are fields that benefit from this technique and especially from this type of apparatus, such as ophthalmology, dentistry, plastic surgery, podiatry, etc.

Thus, when the type of procedure or operation does not require a large magnification (up to 10 x for example), the surgeon can use a simple magnifying glass, which has an individual lens for each eye, integrated into glasses or a headband. This system, although simple and inexpensive, has the disadvantage that the focal length of a magnifying glass cannot be adjusted and, therefore, surgeons or specialists must keep their heads at a specific distance from the operative field, which in many cases makes it unfeasible to have a correct intervention and/or without discomfort for both the patient and the surgeon.

On the other hand, in other less critical areas such as dentistry, it is also known to use image acquisition devices that have magnification capacity and that are placed in one hand while, with the other hand, the necessary medical or surgical tools or instruments are held and manipulated. This type of device, however, makes it difficult for the dentistry to maneuver, since one of the two elements will be handled with the less skilled hand of the doctor. Thus, if the optical device is placed in the less skilled hand and taking into account that these procedures are usually done at awkward angles, blurred images are usually obtained that do not allow to see the details necessary for a correct execution of the procedure. On the other hand, if the optical device is held with the most skilled hand and the instruments with the least skilled one to ensure a good image capture, the integrity of the patient or the proper development of the procedure is put at risk.

In order to solve these problems and free the hand of the doctor or surgeon, devices that integrated the tool or instruments and the camera emerged in the state of the art, that is, the camera was positioned on the tool itself so that the surgeon could only handle one element. An example of this can be seen, for example, in document US 5051823. This solution, however, presents the main problem that the volume of the camera hinders the operation with the tool, at the same time that the tool itself makes vision difficult.

Thus, to solve these problems, this type of device that incorporates cameras has evolved into others that place the camera away from the active tip of the tool so that they do not obstruct each other, as it is the case in the US. 2015/0257636, in which the active tip of the tool is located at a fixed distance in line with the camera lens, the latter being also housed in the device.

These devices, however, even solving the interference problems or difficult handling described above, fall short of expectations in terms of optical performance such as a higher and better quality image magnification capacity.

These magnification needs are currently solved in part by the use of surgical microscopes, which in addition to guaranteeing a great magnification of the image, allow a stable focus, as well as other additional advantages such as better ergonomics, possibility of channeling the image to auxiliary equipment so that they can be seen by a second or third observer, possibility of recording, possibility of projection on screens in real time to use the images for educational/informative and/or pedagogical purposes, etc.

For example, in many complex surgeries surgeons use surgical microscopes to magnify tiny structures such as nerves, blood vessels, tissue injuries, etc. in the operative field, where these equipments are equipped with features that allow the surgeon to focus more on surgery than manipulation of the microscope, such as powered focus and zoom magnification capabilities (usually controlled by a foot pedal), as well as eye tubes, which allow viewing the operative field from above while keeping the head upright. Thus, surgical microscopes enable surgeons in specialties as diverse as dentistry, ophthalmology, otorhinolaryngology, plastic surgery and neurology, to perform complex procedures on delicate structures.

These surgical microscopes are similar to standard laboratory optical binoculars, but are equipped with features that allow them to be used in the surgical environment (e.g., special lighting, relatively large field sizes, etc.). Usually, the body of the microscope itself is supported by an adjustable arm which in turn is attached to a support that can rest on the floor, a wall, the ceiling, etc. Sometimes, in addition, said arm on which the microscope is mounted can also comprise accessories such as a camera or a second microscope, etc.

As has been previously said, these microscopes have in their body an magnification selector, generally a rotating barrel that, when actuated, arranges or places different types of pairs of lenses in the working position, providing different magnifications without altering the focus or the job distance. Thus, the surgeon or specialist can change the magnification ratio using different lenses according to the needs and demands of the task or operation to be performed. For example selecting a 10x magnification for tasks in which a good magnification is needed and a 3x to have less magnification but a broader or overall view.

Schematically, the lens system of these microscopes is made up of the ocular lenses, the magnification barrel and the objectives, the latter being classified according to the working distance, which, as known, it is determined by the focal length (the distance between the objective lens in focus and the object being viewed).

During use, light is directed through prisms or fiber optic cables, and illuminates the field or operation surface through the objective lens, concentric to the field of view. The light beam is reflected from the operative field, through the objective lens and the magnification changer barrel, to the ocular lens and to the place where the surgeon sees the image of the operative field. Sometimes, in addition, a device called a beam splitter is attached, which allows the image to be channeled to photographic or video recording equipment, or to the ocular tube of an assistant or an observer.

Thus, before starting to perform the operation, the corresponding surgeon, doctor or technician must focus the microscope, first by placing the appropriate optical filters, then by turning on the lighting and finally by calibrating the magnification changer. In addition, to achieve clear 3D vision, the distance between the binocular eyepieces shall be adjusted until the two images match, as well as an adjustment to compensate own visual limitations shall be made. Likewise, the surgeon moves the microscope arm until the field of illumination coincides with the field of operation and the view is clearly in focus.

For all of the above, although it is true that these microscopes far exceed the performance of the aforementioned magnifying glasses, of the devices that have to be held with one hand while operating with the other or even those of the devices described above that have a more or less perfected vision or visualization system, they also present important drawbacks that need to be overcome.

Thus, one of these drawbacks is the complex structure and volume they occupy, which results not only in a very high cost that not all fields of medicine or not all those that practice it can afford, but also in discomfort for the surgeon, who may even suffer visual exhaustion and fatigue after prolonged use. Added to this is the disadvantage that, from an ergonomic point of view, it involves having to adopt a posture that is sometimes too tense or upright for a long time, even while sitting.

Said discomfort and postural rigidity is mainly due to the fact that the microscope is calibrated for a certain position in which a correct focus of the part of the patient's body to be operated on is ensured. In other words, the surgeon shall be the one who adapts to the position in which the microscope has been fixed to obtain the most adequate focus according to the previous preparation described above.

For the same reason, the patient shall not be able to move either, as this shall change the distance at which the object to be magnified is located, affecting the magnification and the focus, which also represents a serious discomfort for the patient.

This is undoubtedly an important drawback, to which it has to be added that, for certain operations or actions on the patient, the instruments themselves or the surgeon's hand can become an obstacle that remains in the path of vision and therefore prevent the correct visualization of the work area, which will force it to work "blindly".

To these drawbacks, more related to ergonomics, we must also add an important operational limitation that these types of microscopes have. This limitation is that these devices are limited to displaying an image of the work area, but do not allow real and reliable measurements of the images that are being displayed. Taking into account that the image that is observed is enlarged, there is not even information on dimension and proportion that would be available, due to the surgeon's own experience, in the event that there was no such magnification.

In conclusion, both camera-based and microscope-based systems have drawbacks or defects that prevent their functionality from being complete. Specifically, while camera-based systems do not achieve the magnification or magnification of the image that microscopes achieve, microscopes do not have the autofocus feature that camera-based systems have and force the focus to be given by a fixed distance to the objective.

This, in addition, has the consequence that any of these known systems are difficult to adapt to different branches or different disciplines of medicine or surgical practice, having to select, depending on the case, one or the other and, consequently, assume the deficiencies mentioned before.

For all the above, there is no known system in the state of the art that makes it possible to obtain magnified images with the quality of a microscope, not depending on the focal length and, in addition, to be able to take real and reliable measurements during the operation, being thus applicable to any, or at least to the vast majority of medical disciplines and fields of surgery.

## Description of the invention

The optical system for surgical practice of the invention allows overcoming the drawbacks of the aforementioned state of the art since it allows, due to its structural simplicity and size, an easy and comfortable handling for the doctor or surgeon, which in turn results in a greater comfort for the patient. In addition, it guarantees both a wide range of possibilities of magnification to the correct resolution, which provides enormous versatility in different surgical or medical fields, as well as the ability to perform reliable measurements of the operation area regardless of the degree of magnification that is being used.

Specifically, the optical system of the invention comprises:

- A hollow holding body with an elongated configuration intended to be held or grasped by the doctor, surgeon or specialist to carry out the surgical practice which in turn comprises a digital microscope inside;
- A set of interchangeable tools each equipped, at one end, with removable means of attachment to the holding body and, at the other, with an active tip appropriate for the task to be performed within the field of application: dental, dermatological, podiatric, etc.;
- A display device to process and display the image taken by the digital microscope.

More specifically, the holding body has first and second opposite ends, wherein the first end comprises:

- The lens of the digital microscope; and
- Removable means of attachment of the interchangeable tools for attachment to the holding body such that they allow their locking in at least one specific unequivocal position, preventing relative movement between them and the holding body;

On the other hand, as already mentioned above, unlike digital cameras, which have autofocus, digital microscopes manage their magnification depending on the object-objective distance, so that the smaller this distance, the greater the magnification and therefore they both require the user to focus if the distance to the objective changes.

By virtue of the present invention, it is precisely avoided that the user has to adjust the focus according to the desired degree of magnification.

Specifically, the system of the invention achieves the perfect approach due to the use of a set of interchangeable tools, wherein in each of them, the distance to the free end of the active tip in charge of carrying out the corresponding surgical action is known.

That is, in the system of the invention, the active tip of each of the tools is the objective to focus or focus and achieve the required magnification at that object-objective distance or, even in other words, the active tips will have a pre-designed and known lengths such that they will provide the precise magnification and focus to carry out the activity or operation for which they were designed.

Thus, regardless of the type of work to be carried out, that is, regardless of the medical or surgical field in question, the doctor, surgeon or specialist will not have to worry about the degree of magnification and focus, since selecting the appropriate tool to carry out each work, this tool will already be pre-designed so that its active tip is at the distance (L) from the digital microscope lens that guarantees the correct focus and magnification.

For this reason, as has been previously said, the tools, once coupled to the holding body, remain locked with respect to it, preventing relative movement between them, the position of their active tip constituting a reference position defined by the distance (L) to the lens of the digital microscope housed inside said body. That is to say, the active tips, by maintaining the distance (L) throughout the work, always guarantee the correct magnification and focus of the desired image.

Likewise, the invention also refers, as stated in the claims, to a method for carrying out a surgical practice with image magnification that guarantees a correct operation. Specifically, the method of the invention provides a method to carry out a surgical practice in which magnification is necessary in an easy and comfortable way both for the doctor or surgeon and for the patient, guaranteeing both a wide range of possibilities of magnification to the correct resolution, providing enormous versatility in different medical or surgical fields, such as the ability to make reliable measurements of the surgical site no matter what degree of magnification is being used.

More specifically, the method for carrying out a surgical practice with image magnification comprises the following phases or stages:

- Providing a digital microscope housed inside a holding body intended to be held by the specialist to carry out said surgical practice;
- Providing a display device to process and display the image taken by the digital microscope;
- Providing a set of interchangeable tools, each equipped, at one end, with means of attachment to the holding body that are removable but which allow it to be locked in at least one specific unequivocal position and, at the other end, with an active tip appropriate for the task to be performed within said surgical practice;

Wherein for each of said tools, once attached to the holding body, the distance L from the free end of its active tip to the lens of the digital microscope is known so that each one of them provides the magnification and the precise focus for carrying out the surgical practice for which it has been designed.

Thus, as described above, regardless of the surgical practice that the specialist is going to carry out, the specialist will not have to worry about the degree of magnification and the necessary focus when using each tool, since each of them will be previously designed so that its active tip is at the distance (L) from the digital microscope lens that ensures the correct focus and magnification for that particular practice.

For this reason, as has been previously said, the tools, once engaged to the holding body, remain locked with respect to it, preventing relative movement between them, wherein the position of its active tip constitutes a reference position defined by the distance (L) to the lens of the digital microscope housed inside said body. That is, the active tips, by maintaining the distance (L) throughout the work, always guarantee the correct magnification and focus of the desired image.

Brief description of the drawings

In order to help a better understanding of the features of the invention, according to a preferred example of practical embodiment thereof, a series of drawings is provided as an integral part of said description wherein, with an illustrative and non-limiting nature, the has been represented:

Figure 1.- Shows a schematic view of the optical system of the invention.

Figure 2.- Shows two views, one in elevation 2A of the holding body of the optical system of the invention without an attached tool and another perspective view 2B of the holding body, this time with an interchangeable tool attached to its first end.

Figures 3A - 3H.- Show a set of interchangeable tools belonging to the dental field.

Figures 4A - 4E.- Show a set of interchangeable tools belonging to the podiatry field.

Figures 5A - 5K.- Show a set of interchangeable tools belonging to the dermatology field, plastic surgery and cosmetics.

Detailed description of a preferred embodiment of the invention

One of the embodiments of the optical system for surgical practice of the invention is described below, which is represented in the figures.

Specifically, as can be seen in the figures, the optical system of the invention comprises:

- A hollow holding body (1) of elongated configuration intended to be held or grasped by the doctor, surgeon or specialist to carry out the surgical practice which in turn comprises a digital microscope inside;

As can be seen in the embodiment shown in the figures, this holding body (1) has a cylindrical outer shape, although it can adopt any other ergonomic shape that allows a correct grip, as well as present an outer surface or a coating that prevents it from slipping and escaping from the user's hands.

Likewise, the holding body (1) can be formed by several sections that can be fitted together so that they can be exchanged for others of greater length to facilitate certain treatments, as well as be disassembled or separated into parts to be sterilized without affecting the digital microscope inside or to carry out maintenance and repair tasks of the latter, or even its replacement.

On the other hand, the material used for its manufacture shall be any of a plastic or metallic nature that meets the necessary requirements to be used in medical or surgical practice and, in any case, have sufficient rigidity so that it can be firmly held by the user and guarantee its stability, also serving as an insulator from the heat generated by the digital microscope located inside.

- A set of tools (2) that at one of their ends have means of attachment (not represented in the figures) to be fixed in a removable way to the holding body (1) and, therefore, allow being exchanged between them and at the other end, they have an active tip (3) appropriate for the task to be carried out within the field of application.

As already mentioned, this field can be varied, such as dental, dermatological, podiatric, aesthetic, cleaning, etc.

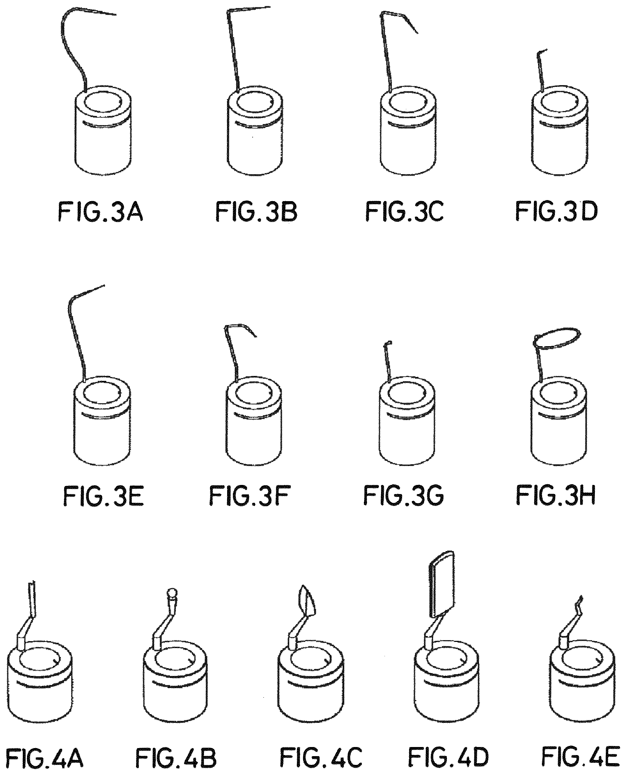

By way of example, in Figures 3A to 3H, there can be seen a set of tools commonly used in dental practice, including a mirror, a curette, a forceps, probes, a condenser, a burnisher, a reamer, etc.

Likewise, also by way of example, in Figures 4A to 4E a set of tools commonly used in podiatry can be seen.

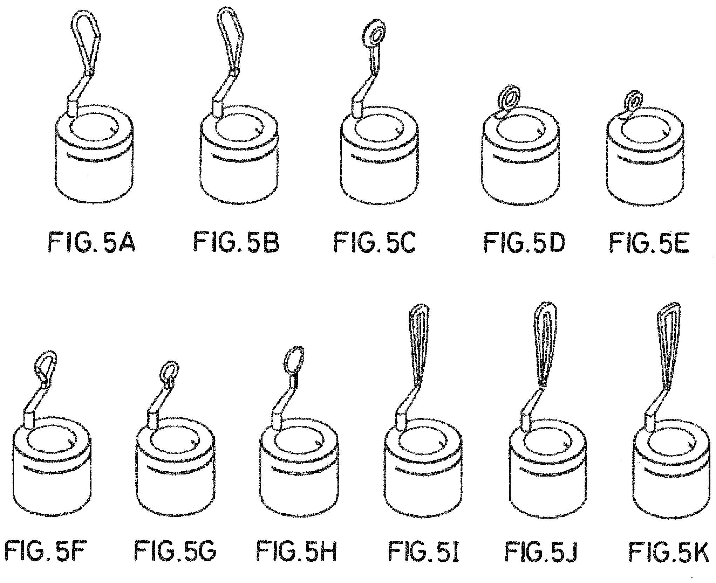

Finally, in Figures 5A to 5K, a set of tools commonly used in dermatology, plastic and cosmetic surgery can be seen, among which there are a curette, a stick, a scalpel, a chisel, a needle, an awl, etc

On the other hand, according to a preferred embodiment of the invention, said active tips (3) comprise one or more marks (3') of known length and that serve as a reference so that the system, as will be explained later, is capable of establishing a measurement scale that can be applied to any other element that appears on the screen to calculate its size.

- A display device (4) to process and display the image taken by the digital microscope once it has been processed.

This display device (4) may be constituted either by the screen of a computer, laptop or desktop, as well as any other mobile device type tablet or telephone, projector or screen of those known today.

Said device will also include the image processor in charge, as its name indicates, of processing the image taken by the digital microscope for its correct visualization.

Likewise, as previously mentioned, the digital microscope can be connected to the display device and, therefore, to the image processor, either by means of cables or wirelessly using Wi-Fi technology, Bluetooth, RFID, either directly or even through an intermediate element.

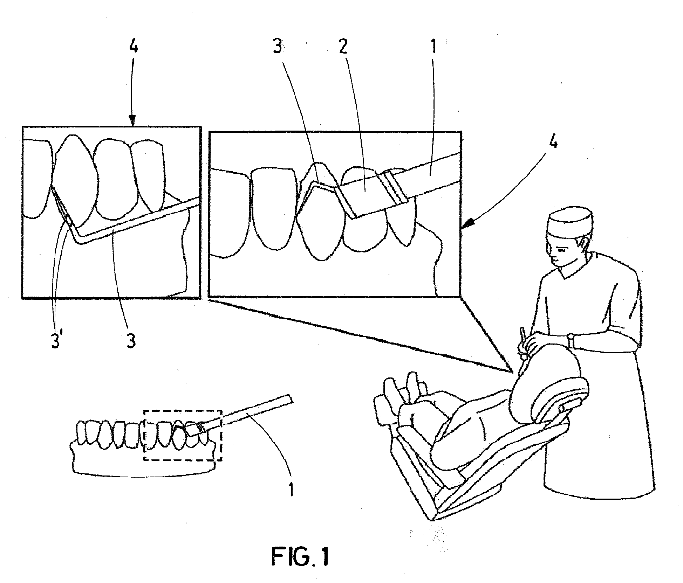

As shown in figure 1, the screen' function is that the doctor, surgeon or specialist follow the operation with the chosen magnification and being able to see the area to be treated or operated not only with said magnification, but also with the focus and sufficient definition to carry it out with the best guarantees. Likewise, this allows the image not only to be seen by the surgeon, but also by the team that supports him, or even by a certain audience such as colleagues, students, family members, etc.

More specifically, the holding body (1) has a first (1') and second (1") ends, wherein:

-    The first end (1') comprises:

     ◦ The lens of the digital microscope, which, although not represented in the figures, is located inside the holding body (1), in correspondence with said first end (1').
     This lens, of course, influences the quality of the image that will be obtained in the display device (4) due to its resolution, which in turn influences the magnification or magnification capacity of the image, as well as the distance to the objective and the size of the display device screen (4), as will be seen later.
     ◦ Means of attachment complementary to the removable means of attachment of the tools (2) that, as already said, allow the locking of said tools (2) in a certain position, preventing relative movement between them and the holding body (1).
     The way to achieve said locking may be through any conventional coupling mechanism, for example, threading, dovetail construction, clipping, friction, etc. In any case, any of these means shall guarantee that, in addition to the fact that the tools (2) can be attached to the holding body (1) and can be easily removed, once they are attached there is no relative movement between them.
     However, according to a possible preferred embodiment of the invention, instead of a single unequivocal position, the means of attachment of the tools (2) in correspondence with the means of attachment of the holding body (1) may have more than one locking position, preferably 4, each coinciding with the positions at 0°, 90°, 180° and 270°. In this way, it shall be allowed that the active tip (3) be placed in 4 positions that cover a round turn at 90° intervals, thus allowing working in difficult or compromised access condition.

In other words, in said embodiment, the attachment between the tool (2) and the holding body (1) can be carried out in four different positions, thus allowing greater versatility.

Thus, for example, in the event that the doctor, surgeon or specialist has some difficulty in how to handle the active tip (3) of the tool (2), for example in the mouth of a patient, she/he can release or unblock said tool (2), turn it to one of the other positions and attach it again to the holding body (1) to continue working more comfortably.

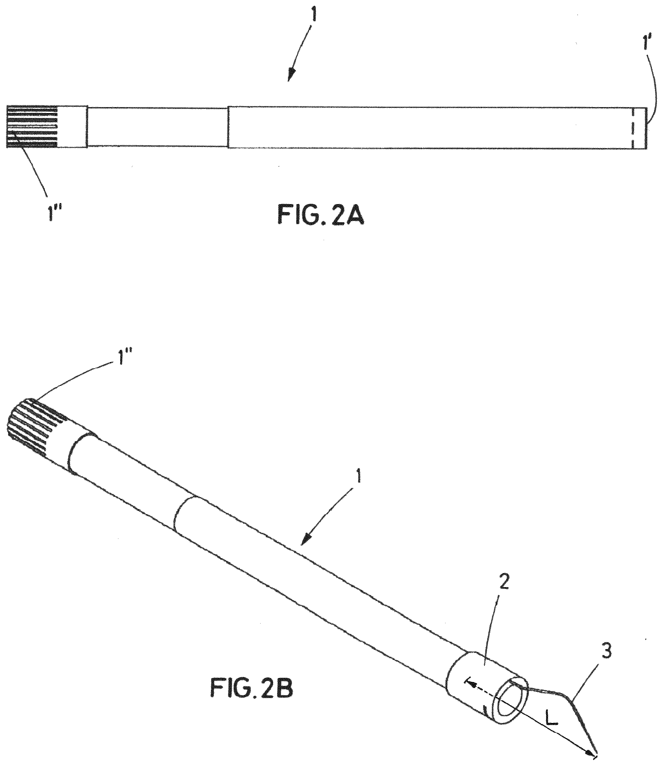

-    The second end (1"), which is opposite the first one, as can be seen in Figures 1 and 2, is the place where the power supply cables of the digital microscope and/or the communication cables of the digital microscope with the display device and/or image processor or with a storage device, come out, and/or in the event that wireless technology is not used and the power supply is not by means of batteries, this is also possible.

On the other hand, the following describes more specifically how the system of the present invention carries out in a controlled, reliable and simple way the magnification or enlargement of the image captured by the digital microscope as a function of the object-objective distance, wherein, as is known in the state of the art, the smaller this distance, the greater the magnification, and it is necessary to modify the focus each time it is changed.

Thus, thanks to the present invention, it is precisely avoided that the user has to adjust the focus as a function of the desired degree of magnification.

Specifically, the system of the invention shown in the figures achieves the perfect approach by virtue of the use of the set of interchangeable tools (2), wherein for each of them the distance or length (L) to the free end of the active tip (3) is known and, therefore, to the lens in charge of carrying out the corresponding surgical procedure.

In other words, the active tip (3) of each of the tools (2) is the objective to focus and achieve the required magnification at that object-objective distance.

Given that once the tools (2) are engaged to the holding body (1), they remain fixed and without the possibility of relative movement between them and that the distance or length (L) of the active tip (3) of the tools (2) to the lens is known, therefore the object-objective distance is known throughout the duration of the work with the same tool (2).

Therefore, the present invention presents the active tip (3) of each of the tools (2) as the first objective to focus and achieve the required magnification or enlargement.

In other words, the active tip (3) of the tool (2) is in a reference position, where said reference position is defined by the distance or length (L) from the active tip (3) to the lens located at the end of the clamping body (1), that is, the active tips (3), by maintaining the distance or length (L) throughout the work, always guarantee the desired magnification.

Likewise, as already mentioned, the invention also refers to a method for carrying out a surgical practice with image magnification that guarantees a correct operation or intervention by the specialist with regard to magnification and focus of the tool (2).

Specifically, a preferred embodiment of the method for carrying out a surgical practice with image magnification comprises the following phases or stages:

- Providing a digital microscope housed inside a holding body (1) intended to be held by the specialist to carry out said surgical practice;
- Providing a display device (4) to process and display the image taken by the digital microscope;
- Providing a set of interchangeable tools (2) each equipped, at one end, with means of attachment to the holding body (1) that are removable but allow it to be locked in at least one specific unequivocal position and, at the other end, with an active tip (3) appropriate for the task to be performed within said surgical practice;

Wherein, as can be seen in Figure 2B, for each of said tools (2), once attached to the holding body (1), the distance or length L from the free end of its active tip (3) to the digital microscope lens (not shown) is known so that each lens provides the precise magnification and focus required to perform the surgical practice for which it was designed.

Thus, as described above, regardless of the surgical practice that the specialist is going to carry out, he or she will not have to worry about the degree of magnification and the necessary focus when using each tool (2), since each of them will be previously designed so that its active tip (3) is at the distance or length (L) from the digital microscope lens that guarantees the correct focus and magnification for that particular practice.

For this reason, as has been said, the tools (2), once engaged to the holding body (1), remain locked with respect to it, preventing relative movement between them, the position of their active tip (3) constituting a position reference defined by the distance or length (L) to the lens of the digital microscope housed inside said holding body (1). This means that the active tips, by maintaining the distance or length (L) throughout the work, always guarantee the correct magnification and focus of the desired image.

However, the method of the invention also contemplates providing several unambiguous locking positions between the means of attachment of the tools (2) and the corresponding means of attachment of the holding body (1), preferably 4, each one coinciding with the positions at 0°, 90°, 180° and 270°, maintaining in all of them the same distance or length L between the active tip (3) and the lens of the digital microscope. In this way, it will be possible that the active tip (3) of the tools (2) can be placed in 4 positions that cover a 360° turn at 90° intervals, thus allowing work under difficult or compromised access conditions under the same conditions of magnification and focus.

For example, in a possible embodiment of the method of the invention, a set of tools (2) is provided for dental practice such as those shown in Figure 1, so that the dimension of said tools is known and varies depending on the specific task for which each of them are used. Thus, once a specific tool (2) has been engaged to the holding body (1) and in the locking position with respect to it, the distance or length (L) from the active tip (3) of said tool (2) to the lens of the digital microscope is also known.

For example, if said distance or length to the lens is 14mm, a magnification or enlargement of approximately 34x is obtained at the active tip (3) with optimal focus. According to another embodiment, if the active tip (3) of the tool (2) is located at a distance or length L of 7 mm from the lens of the digital microscope, a magnification or enlargement of approximately 68x is obtained at said active tip (3) and also an optimal focus.

However, as also mentioned above, the magnification not only depends on the aforementioned distance or length L, but also on the resolution of the digital microscope lens, and on the size and resolution of the screen of the display device (4).

Specifically, the relationship between these variables can be defined with the following formula:

$$\text{Magnification} = \frac{\left(\dfrac{Rm}{Rp}\right) \times Tp}{L}$$

Wherein:

Rm = Horizontal resolution of the digital microscope lens (in dots or pixels).
Rp = Horizontal resolution of the display device screen (4) (in dots or pixels).
Tp = Size of the screen of the display device (4) (in cm).
L = Object - Objective Distance (in cm) = length L from the digital microscope lens to the active tip (3).

Thus, for example, applying that formula to the specific case in which you have:
A digital microscope whose lens has a resolution of 2592 horizontal dots or pixels and 1280 vertical dots or pixels; therefore Rm = 2592 dots or pixels;

A screen of the display device (4) with a size of 14" and a resolution of 1920 horizontal dots or pixels and 1024 vertical dots or pixels; therefore Tp = 14" x 2.5cm = 35cm and Rp = 1920 pixels;

For three different types of interchangeable tools (2), let suppose that they are from the field of dentistry, for example those indicated in figures 3E, 3F and 3G, and assuming lengths L from their active tips to the microscope lens of 1.4cm , 0.7cm and 0.5cm respectively, the following magnification or enlargement table is obtained.

| L (in cm) | 1.4 | 0.7 | 0.5 |
|---|---|---|---|
| Approximate magnification | 34x | 68x | 95x |

Finally, the method of the invention also allows, as already indicated initially, taking real and reliable measurements during the operation, treatment or surgical practice, which, obviously, is extremely useful leading to an optimal result.

For this, once the surgeon, technician or specialist has mounted a specific tool (2) in the holding body (1) and locked both by means of the means of attachment in a certain position, the method of the invention allows taking said measurement by following the steps below:

- Capturing an image with the digital microscope in which the active tip (3) of the tool (2) appears.
- Sending the image captured in the previous step to a display device (4) and showing it on its screen; By means of an image processing software, capturing the image captured in the previous steps and selecting from said captured image shown on the screen of the display device (4) two first points separated from each other by a known distance to establish the scale.

    Said two points could be, for example, the width of the active tip (3) or any other of its known dimensions or, according to another possible embodiment, one or more visible marks (3') and of known length and dimensions such that they can serve as a reference for the system, for example, parallel lines, perpendicular lines, geometric figures such as squares, triangles, circles, rectangles, pentagons, etc.

For example, in the event that said marks (3') are formed, as in the example shown in figure 1, by two parallel lines, the known distance between them can be counted by the image processing software either automatically when identifying and measuring the aforementioned marks (3') as the points to establish the scale, or only by identifying said marks (3') but asking the user to manually enter the distance through any interface, in which said value could be entered into the system at any time, even one prior to capturing the image.

- Once the scale of the image captured by the previous step has been established, by means of the image processing software, selecting in the captured image shown on the screen of the display device (4) two second points for which y the distance therebetween has to be known.
- Calculating, by means of the image processing software, the real measurement that separates the two second points as a result of establishing the real measurement to which a pixel on the screen corresponds using the distance between the first two points as a standard for, later, multiplying said actual measurement to which a pixel on the screen corresponds by the distance in pixels existing between the two second selected points; and
- By means of the image processing software, showing on the screen of the display device (4) the real measurement that separates the two second points calculated in the previous step,

Thus, an example of the application of the measurement method is exemplified in figure 1, wherein there can be seen two examples of image captures that the specialist can observe on the screen of the display device (4), in this case corresponding to the dental field. In one of them, the one with largest scale, it can be seen how the active tip (3) of the tool (2) is superimposed on the patient's teeth.

Specifically, in this case, the scale reference of said marks (3') are parallel lines marked on the active tip of the tool (3) separated by a predetermined distance.

Thus, for a specific example in which the two parallel lines that make up said marks (3') were separated by a real distance of 1 mm, this would mean that if the distance in the image corresponds to 10 pixels, then any pair of points found on the screen, for example two ends of a tooth, which are 50 pixels apart, their actual distance will be 5mm.

**Claims**

1. Optical system for surgical practice comprising:

    - A hollow holding body (1) with an elongated configuration which in turn comprises a digital microscope inside;
    - A set of tools (2) which in turn comprise removable means of attachment to the holding body (1) and an active tip (3).
    - A display device (4) with an image processor for digital processing of the image taken by the digital microscope and a screen for displaying said image;

    **characterized in that**

- the holding body (1) comprises first (1') and second (1'') ends, such that the first end (1') comprises the lens of the digital microscope and means of attachment complementary to the removable means of attachment of the tools (2) that allow locking without relative movement of both elements to each other; and **in that**
- once a tool (2) has been fixed on the holding body (1), its active tip (3) is located at a length L from the lens such that the digital image obtained by the digital microscope is focused on said active tip (3) for each given magnification.

2. Optical system for surgical practice according to claim 1, **characterized in that** the means of attachment of tools (2) in correspondence with the means of attachment of the holding body (1) comprise different locking positions.

3. Optical system for surgical practice according to claim 2, **characterized in that** the means of attachment of the tools (2) to the holding body (1) comprise four different locking positions 90° apart.

4. Optical system for surgical practice according to any of the preceding claims, **characterized in that** the holding body (1) has a cylindrical outer shape.

5. Optical system for surgical practice according to any of the preceding claims, **characterized in that** the holding body (1) is formed by several sections capable of being fitted together.

6. Optical system for surgical practice according to any of the preceding claims, **characterized in that** the active tips (3) comprise at least one mark (3') of known length.

7. Optical system for surgical practice according to any of the preceding claims, **characterized in that** the second end (1") of the holding body (1) constitutes the outlet for the electrical power supply cables of the digital microscope and/or the communication cables of the digital microscope with the display device or with a storage device.

8. Optical system for surgical practice according to any of the preceding claims 1 to 5, **characterized in that** the digital microscope of the holding body (1) uses wireless technology for communication with the display device or with a storage device and **in that** its electrical supply is carried out by batteries.

9. Method for carrying out a surgical practice with image magnification **characterized in that** it comprises:

- Providing a digital microscope housed inside a holding body (1) intended to be held by the specialist to carry out said surgical practice;
- Providing a display device (4) to process and display the image taken by the digital microscope;
- Providing a set of interchangeable tools (2) each equipped, at one end, with means of attachment to the holding body (1) that are removable but allowing it to be locked in at least one specific unequivocal position and, in the other end, with an active tip (3) appropriate for the task to be performed within said surgical practice;

wherein for each of said tools (2), once fixed to the holding body (1), the length L from the free end of its active tip (3) to the lens of the digital microscope is known so that each of them provide the precise magnification and focus to carry out the surgical practice for which it was designed.

10. Method for carrying out a surgical practice with image magnification according to claim 9, **characterized in that** once a certain tool (2) has been mounted on the holding body (1) and both of them are locked by means of attachment in a certain position, it comprises:

- Capturing an image with the digital microscope in which the active tip (3) of the tool (2) appears.
- Sending the image captured in the previous step to a display device (4) and displaying it on its screen;
- By means of an image processing software, capturing the image captured in the previous steps and selecting in said captured image shown on the screen of the display device (4) two first points separated from each other by a known distance to establish the scale.
- Once the scale of the image captured by the previous step has been established by means of the image processing software, selecting in the captured image shown on the screen of the display device (4) two second points for which the distance therebetween has to be known.
- Calculating, using the image processing software, the real measurement that separates the two second points as a result of establishing the real mean to which a pixel on the screen corresponds, using the distance between the first two points as a standard for, later, multiplying said real measurement to which a pixel on the screen corresponds to the distance in pixels existing between the two second selected points; and
- By means of the image processing software, showing on the screen of the display device (4) the real measurement that separates the two second points calculated in the previous step,

**11.** Method for carrying out a surgical practice with image magnification according to claim 10, **characterized in that** the first two points separated from each other by a known distance are made up of one or more visible marks (3') that the active tip (3) of the tool (2) has.

**12.** Method for carrying out a surgical practice with image magnification according to claim 11, **characterized in that** the marks (3') are two parallel lines.

**13.** Method for carrying out a surgical practice with image magnification according to any of claims 9 to 12, **characterized in that** the distance between the first two points separated from each other is automatically counted by the image processing software when identifying and measuring the aforementioned marks (3') as the points to establish the scale.

**14.** Method for carrying out a surgical practice with image magnification according to any of claims 9 to 12, **characterized in that** the distance between the first two points separated from each other is provided to the image processing software manually by the user.

**15.** Method for carrying out a surgical practice with image magnification according to any of claims 9 to 14, **characterized in that** the means of attachment of the tools (2) and the corresponding means of attachment of the holding body (1) comprise four unequivocal positions 90° apart from each other so that for the same tool (2) all of them maintain the same length L between the active tip (3) of said tool (3) and the lens of the digital microscope.

FIG.1

1

1'

1"

FIG.2A

1"

1

2

3

L

FIG.2B

FIG.3A   FIG.3B   FIG.3C   FIG.3D

FIG.3E   FIG.3F   FIG.3G   FIG.3H

FIG.4A   FIG.4B   FIG.4C   FIG.4D   FIG.4E

FIG.5A  FIG.5B  FIG.5C  FIG.5D  FIG.5E

FIG.5F  FIG.5G  FIG.5H  FIG.5I  FIG.5J  FIG.5K

Europäisches
Patentamt

European
Patent Office

Office européen
des brevets

# EUROPEAN SEARCH REPORT

Application Number

EP 20 38 2695

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 743 731 A (LARES CRAIG J [US] ET AL) 28 April 1998 (1998-04-28) | 1-5,7,9, 15 | INV. A61B1/24 |
| Y | * columns 2-9 - pages 1-4 * | 2,3,6-8, 10-15 | A61B90/20 A61B90/00 |
| | ----- | | A61C3/00 |
| X | DE 198 49 660 A1 (SCHEPPERHEYN HANS PETER [DE]) 11 May 2000 (2000-05-11) | 1,4,5,7, 9 | G02B21/00 A61B1/00 |
| Y | * paragraphs 1, 6-24, 30-33 - pages 1-4 * | 2,3,6-8, 10-15 | G02B25/00 A61C19/00 |
| | ----- | | G06T7/00 |
| X | US 2019/328221 A1 (WINK CHERIE YVETTE [US] ET AL) 31 October 2019 (2019-10-31) | 1,4,5,8, 9 | |
| Y | * paragraphs [0002], [0008], [0017] - [0028]; figures 1-7 * | 2,3,6-8, 10-15 | |
| | ----- | | |
| A | US 2005/043588 A1 (TSAI JORY [US]) 24 February 2005 (2005-02-24) * the whole document * | 1-15 | |
| | ----- | | |
| A | EP 0 821 907 A1 (LARES RESEARCH INC [US]) 4 February 1998 (1998-02-04) * the whole document * | 1-15 | TECHNICAL FIELDS SEARCHED (IPC) |
| | ----- | | A61B |
| Y | EP 1 478 296 A1 (DEPUY INT LTD [GB]) 24 November 2004 (2004-11-24) * paragraphs [0032] - [0036]; figures 1, 2 * | 6,10-14 | A61C G02B G06T |
| | ----- | | |
| Y | JP 2009 268614 A (ADVANCE CO LTD) 19 November 2009 (2009-11-19) * paragraphs [0055] - [0075]; figures 17-19 * | 6,10-14 | |
| | ----- | | |
| Y | US 5 224 049 A (MUSHABAC DAVID R [US]) 29 June 1993 (1993-06-29) * column 12 - column 13; figures 7, 9, 10 * | 10-14 | |
| | ----- | | |

The present search report has been drawn up for all claims

| Place of search | Date of completion of the search | Examiner |
|---|---|---|
| Munich | 23 February 2021 | Knorn, Raphaela |

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 20 38 2695

The Search Division considers that the present European patent application does not comply with the requirements of unity of invention and relates to several inventions or groups of inventions, namely:

1. claims: 1-5, 7-9, 15

   Composition of an optical system for surgical practice, comprising a holding body with an integrated digital microscope and an interchangeable set of reversibly attachable tools. Details concerning the structural features of said system.

   ---

2. claims: 6, 10-14

   Calibration of the measurement scale for an optical dental or surgical instrument, comprising an imaging sensor, processing means, a display screen and at least one dental/ surgical tool with an active tip, comprising a reference marking.

   ---

## ANNEX TO THE EUROPEAN SEARCH REPORT
## ON EUROPEAN PATENT APPLICATION NO.

EP 20 38 2695

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2021

| Patent document cited in search report | | Publication date | Patent family member(s) | | Publication date |
|---|---|---|---|---|---|
| US 5743731 | A | 28-04-1998 | NONE | | |
| DE 19849660 | A1 | 11-05-2000 | NONE | | |
| US 2019328221 | A1 | 31-10-2019 | JP | 2020500047 A | 09-01-2020 |
| | | | US | 2019328221 A1 | 31-10-2019 |
| | | | WO | 2018080934 A1 | 03-05-2018 |
| US 2005043588 | A1 | 24-02-2005 | NONE | | |
| EP 0821907 | A1 | 04-02-1998 | EP | 0821907 A1 | 04-02-1998 |
| | | | US | 5634790 A | 03-06-1997 |
| EP 1478296 | A1 | 24-11-2004 | AT | 538747 T | 15-01-2012 |
| | | | AU | 2003212505 A1 | 09-09-2003 |
| | | | AU | 2003212506 A1 | 09-09-2003 |
| | | | EP | 1478296 A1 | 24-11-2004 |
| | | | EP | 1478297 A1 | 24-11-2004 |
| | | | JP | 4387200 B2 | 16-12-2009 |
| | | | JP | 2005518245 A | 23-06-2005 |
| | | | JP | 2005518246 A | 23-06-2005 |
| | | | US | 2005203544 A1 | 15-09-2005 |
| | | | US | 2005251186 A1 | 10-11-2005 |
| | | | US | 2011082460 A1 | 07-04-2011 |
| | | | WO | 03071968 A1 | 04-09-2003 |
| | | | WO | 03071969 A1 | 04-09-2003 |
| JP 2009268614 | A | 19-11-2009 | NONE | | |
| US 5224049 | A | 29-06-1993 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5051823 A
- US 20150257636 A